# EUROPÄISCHE PATENTSCHRIFT

(11) **EP 2 120 953 B1**
(45) Veröffentlichungstag und Bekanntmachung des Hinweises auf die Patenterteilung: **19.09.2018**
(21) Anmeldenummer: 07856544.7
(22) Anmeldetag: 11.12.2007
(51) Int. Cl.: A61K 31/505, A61K 31/164, A61K 31/17, A61K 31/122, A61K 36/38, A61P 17/06, A61P 17/00, A61K 9/00

(54) **ZUBEREITUNGEN FÜR DIE TOPISCHE BEHANDLUNG VON HAUTERKRANKUNGEN**
PREPARATIONS FOR THE TOPICAL TREATMENT OF SKIN DISEASES
COMPOSITION POUR LE TRAITEMENT TOPIQUE DE MALADIES CUTANÉES

(30) Priorität: 20.03.2007 DE 102007013857
(43) Veröffentlichungstag der Anmeldung: 25.11.2009
(73) Patentinhaber: Maria Clementine Martin Klosterfrau Vertriebsgesellschaft mbH, 50670 Köln (DE)
(72) Erfinder: VESTWEBER, Anna-Maria, 51399 Burscheid (DE); BIEGERT, Thomas, 50968 Köln (DE)
(74) Vertreter: Von Rohr Patentanwälte Partnerschaft mbB
(86) Internationale Anmeldenummer: PCT/EP2007/010791
(87) Internationale Veröffentlichungsnummer: WO 2008/113400

(56) Entgegenhaltungen:
- WO-A-2005/002581
- WO-A-2005/063266
- WO-A-2006/018198
- DE-A1-102004 016 129
- DE-A1-102004 049 062
- FR-A- 2 877 222

## Beschreibung

Die vorliegende Erfindung betrifft den Bereich der Hautkrankheiten (Dermatosen).

Insbesondere betrifft die vorliegende Erfindung eine Zusammensetzung, insbesondere eine pharmazeutische Zusammensetzung, zur Behandlung von Hauterkrankungen. Bei den Erkrankungen handelt es sich insbesondere um atopische Dermatitis oder Psoriasis. Die Zusammensetzung nach der Erfindung enthält in Kombination Ectoin bzw. mindestens ein Ectoinderivat zusammen mit Harnstoff bzw. mindestens einem Harnstoffderivat und/oder mindestens einem Inhaltsstoff des Johanniskrauts *(Hypericum perforatum L.).*

Des weiteren betrifft die vorliegende Erfindung ein Behältnis, welches die erfindungsgemäße Zusammensetzung enthält.

Schließlich betrifft die vorliegende Erfindung die Verwendung der Zusammensetzung nach der Erfindung im Bereich der Pharmazie, Medizin oder Kosmetik sowie zur prophylaktischen oder therapeutischen Behandlung von Hauterkrankungen, wie atopischer Dermatitis oder Psoriasis.

Die Haut (*Cutis*) ist das den Organismus bzw. Körper bedeckende oberflächengrößte Organ. Sie dient dem physikalischen, chemischen und immunologischen Schutz, der Wärmeregulation und der Aufnahme von Sinnesreizen. Die Haut fungiert gewissermaßen als Grenzorgan bzw. Grenzfläche des Organismus zu seiner Umgebung. Kein anderes Organ des menschlichen Körpers weist eine so hohe Zahl an krankhaften Veränderungen auf wie die Haut. Viele Hautkrankheiten können anhand visuell wahrnehmbarer Veränderungen bestimmter Hautareale wahrgenommen bzw. diagnostiziert werden. Derartige Veränderungen der Haut werden im allgemeinen auch als sogenannte Effloreszenzen bezeichnet.

Zu den am häufigsten verbreiteten Hautkrankheiten zählt die Schuppenflechte, welche medizinisch auch als Psoriasis bezeichnet wird. Die Psoriasis kann aufgrund von stark schuppenden, punktförmigen bis handtellergroßen Hautstellen, welche häufig an den Knien, den Ellenbogen und der Kopfhaut auftreten, erkannt werden. Als Ursache werden Entzündungsreaktionen der Haut verantwortlich gemacht, die ein überschießendes Wachstum von hautbildenden Zellen (Keratinozyten) bewirken, wobei die Entzündungsreaktionen oftmals durch eine fehlgeleitete Immunreaktion hervorgerufen werden. Die befallenen Stellen jucken mitunter stark, wobei der Juckreiz sehr störend sein kann, und die stark schuppenden Hautstellen sind kosmetisch nachteilig. Im allgemeinen ist die psychische Belastung der betroffenen Patienten sehr groß.

Zur Behandlung der Psoriasis wird im Stand der Technik im Rahmen einer topischen Applikation oftmals Harnstoff als Monopräparat in Form von Beigaben zu Ölen, Cremes bzw. Salben eingesetzt, wobei der Behandlungserfolg jedoch nicht immer zufriedenstellend ist. Weiterhin wird im Stand der Technik oftmals Salicylsäure topisch auf die befallenen Hautstellen appliziert, um ein Ablösen der Schuppen zu ermöglichen. Auch hier ist der Therapieerfolg nicht immer ausreichend. Zudem wird seit langer Zeit zur Behandlung chronischer Hauterkrankungen, wie Psoriasis, Steinkohlenteer verwendet, welcher die Zellteilung bremst und den Juckreiz lindert. Allerdings gelten die verwendeten Teerstoffe inzwischen als krebsfördernd, weswegen sie nur noch eingeschränkt eingesetzt werden. Eine weitere Behandlungsmöglichkeit der Psoriasis besteht in dem Einsatz von Dithranol bzw. Cignolin, welches die Zellteilung vermindert. Der Nachteil dieser Methode besteht darin, daß die Behandlung extrem aufwendig ist und oftmals eine unerwünschte Verfärbung der angrenzenden gesunden Haut resultiert. Auch werden oftmals Vitamin-D-Derivate eingesetzt, wobei derartige Medikamente bei Überdosierung gefährliche Nebenwirkungen aufweisen können. Schließlich wird im Rahmen der Behandlung von Psoriasis oftmals Cortison lokal appliziert, wobei aufgrund der starken Nebenwirkungen von Cortison dieses jedoch nur kurzzeitig und auf kleine Hautareale eingesetzt werden kann.

Eine weitere, häufig verbreitete Hautkrankheit ist das sogenannte atopische Ekzem, dessen Hauptsymptome rote, schuppende, manchmal auch nässende Ekzeme auf der Haut und ein oftmals quälender Juckreiz sind. Das atopische Ekzem wird synonym auch als Neurodermitis, atopische Dermatitis und endogenes Ekzem bezeichnet.

Das atopische Ekzem bzw. die Neurodermitis gilt als nicht heilbar, ist aber in gewissem Maße behandelbar. Die Bereitschaft, eine atopische Dermatitis zu entwickeln, ist bei 5 bis 20 % der Kinder und bei 1 bis 3 % der Erwachsenen vorhanden. Die Symptome äußern sich bei jedem Betroffenen in unterschiedlicher Ausprägung und an verschiedenen Stellen. Die Erkrankung tritt meist in Schüben von unterschiedlicher Dauer und Stärke auf.

Das Hauptproblem für die Betroffenen ist bei der Neurodermitis - vergleichbar zur Psoriasis - der starke Juckreiz. Bei den Betroffenen tritt auch häufig eine starke psychische Belastung insbesondere aufgrund des starken Juckreizes und der mit einer Kratzaktivität einhergehenden Wunden auf. Neurodermitis äußert sich insbesondere durch eine sehr empfindliche und trockene Haut, die auch oft gerötet ist. In Verbindung mit dem Juckreiz und dem starken Bedürfnis, auf diesen mit Kratzen zu reagieren, kann die Haut rissig werden, wodurch Ekzeme entstehen. Typische Stellen für die von der Krankheit betroffene Haut sind insbesondere die Armbeugen, die Kniekehlen sowie die Hals- und Gesichtspartie.

Die sehr empfindliche und oft rissige Haut eines von Neurodermitis betroffenen Patienten ist zudem anfällig gegenüber Infektionen, insbesondere was die teils massive Besiedlung der betroffenen Hautstellen mit *Staphylococcus aureus* anbelangt. Diese Bakterien führen durch Auslösung nässender Hautreaktionen zu einer zunehmenden Verschlechterung des Hautzustandes, was für den Betroffenen äußerst nachteilig ist.

Die Ursachen der Neurodermitis sind bislang nicht eindeutig geklärt. Es wird jedoch davon ausgegangen, daß die Betroffenen aufgrund genetischer Veranlagungen stärker auf bestimmte Einflüsse reagieren als andere. Neuere Untersuchungen zeigen, daß bei Neurodermitikern eine Gensequenz beschädigt ist, die für ein Enzym kodiert, welches für die Produktion von Hautfetten verantwortlich ist. Durch die verminderte bzw. nicht vorhandene Hautfettsynthese trocknet die Haut schnell aus und wird rissig, was die Neurodermitis hervorrufen bzw. weiter verschlimmern kann. Hinweise bestehen auch in bezug auf eine Involvierung des vegetativen Nervensystems. Dieser Aspekt erklärt, warum Betroffene bei Streß zu Ausschlagsschüben neigen.

Im Stand der Technik werden zur Behandlung der Neurodermitis diverse Medikamente in Form von Salben, Cremes und Lotionen vorgeschlagen.

Wie bei der Psoriasis werden zur Linderung der Neurodermitis vielfach Cortison bzw. Cortisonpräparate eingesetzt. Eine derartige Therapie ist jedoch mit zahlreichen Nebenwirkungen verbunden, zu denen eine Hautverdünnung, starke Behaarung, Dehnungsstreifen sowie eine partielle Unterdrückung des örtlichen Immunsystems zählen. Bei längerer Anwendung von stark wirkenden Präparaten können - trotz der topischen Applikation - sogar Nebenwirkungen auftreten, die den gesamten Organismus betreffen. Zudem ist problematisch, daß die krankheitsbedingten Hautveränderungen nach Absetzen der cortisonhaltigen Präparate häufig erneut auftreten, so daß die Betroffenen wieder mit Cortison behandelt werden müssen. Zudem tritt bei Cortisonpräparaten häufig eine sogenannte Tachyphylaxie auf, d. h. eine Wirkabschwächung der Präparate mit zunehmendem Gebrauch. In jüngerer Zeit werden als immunsuppressiv wirkende Substanzen auch spezielle Vertreter der Gruppe der Makrolide eingesetzt. Diese weisen geringere Nebenwirkungen als cortisonhaltige Präparate auf, jedoch ist der Therapieerfolg nicht immer ausreichend. Zudem kommt es in einigen Fällen zu nicht unerheblichen Nebenwirkungen, wie Hautinfektionen und Unverträglichkeitsreaktionen.

Als weitere Wirkstoffe stehen Gerbstoffpräparate, die sogenannten Tannine, zur Verfügung, die leicht antientzündlich wirken. Zudem kommen Präparate von Schieferöl, wie Leukichthyol, zum Einsatz. Nachteilig ist hier, daß auch moderne Zubereitungen nicht geruchsneutral sind.

Auch im Rahmen der Neurodermitisbehandlung wird oftmals Harnstoff eingesetzt, da die Haut von Neurodermitiskranken eine signifikant niedrigere Konzentration von Harnstoff aufweist, welcher als wichtiger Feuchthaltefaktor fungiert. Der Einsatz von Harnstoff als Monopräparat ist jedoch nicht immer erfolgsversprechend, insbesondere kann es bei Verwendung von Präparaten mit hoher Harnstoffkonzentration, wie sie im Rahmen von Monopräparaten vorgesehen sein kann, bei der ohnehin schon gereizten oder rissigen Haut zu weiteren Reizungen kommen.

Die EP 0 887 418 B1 betrifft die Verwendung von Ectoin und Hydroxyectoin zur Herstellung eines Arzneimittels zur Behandlung von Hauterkrankungen. Spezifische Hauterkrankungen werden nicht genannt, und das Dokument zielt vorrangig auf ein spezielles Herstellungsverfahren der Wirksubstanz ab. Das in diesem Dokument beschriebene Monopräparat weist zudem eine nicht immer optimale Wirkung auf.

Weiterhin betrifft die EP 1 131 063 B1 die Verwendung einer Salbe bzw. Creme, welche als Wirksubstanz Hyperforin bzw. Hypericin enthält, zur Behandlung spezifischer Hauterkrankungen. Auch hierbei handelt es sich um ein Monopräparat, dessen Wirkung in bezug auf die angesprochenen Hauterkrankungen nicht immer erfolgsversprechend ist.

Die WO 2006/018198 A1 betrifft topische kosmetische oder dermatologische Zusammensetzungen, die in einem geeigneten kosmetischen oder dermatologischen Träger eine Kombination mindestens eines alkyl- oder hydroxyalkylsubstituierten Harnstoffes mit mindestens einem ausgewählten kosmetischen oder dermatologischen Wirkstoff enthalten. Neben einer großen Vielzahl an kosmetischen Wirkstoffen, wie Aminosäurederivaten, DNA- oder RNA-Oligonucleotiden, Betainverbindungen, Vitaminen oder dergleichen, kann es auch vorgesehen sein, daß die dort beschriebene Zusammensetzung Ectoin enthält.

Die FR 2 877 222 A1 betrifft eine kosmetische Zusammensetzung, welche ein feuchtigkeitsspendendes Mittel sowie einen Hydroxyalkylharnstoff aufweist.

Die WO 2005/063266 A1 beschreibt therapeutische Zusammensetzungen zur Behandlung von Dermatosen, wobei die Zusammensetzungen einen Extrakt von *Calendula officinalis* und *Hypericum perforatum* aufweisen.

Weiterhin betrifft die WO 2005/002581 A1 die Verwendung von Osmolyten, insbesondere Ectoin und Hydroxyectoin, sowie von deren pharmakologisch verträglichen Salzen und/oder gleichwirkenden Derivaten zur Herstellung von dermatologischen Zubereitungen, wie Tinkturen, Lotionen, ONV-Emulsionen, W/O-Emulsionen, Cremes, Salben, Hydrogelen oder Sprays, zur topischen Prophylaxe, Pflege und/oder Behandlung der Neurodermitis.

Die DE 10 2004 016 129 A1 und die DE 10 2004 049 062 A1 betreffen zudem jeweils die Verwendung von Osmolyten aus extremophilen Mikroorganismen in Kombination mit Öl der Nachtkerze (*Oenothera biennis*) (Oleum Oenotherae) zur Herstellung von Zubereitungen zur topischen Anwendung in der Kosmetik und Dermatologie zur Pflege sehr trockener, irritierter, schuppiger und problematischer Haut.

Insgesamt führen die im Stand der Technik vorgesehen medikamentösen Ansätze zur Behandlung von Hauterkrankungen, insbesondere der Psoriasis bzw. des atopischen Ekzems, nicht immer zu einem ausreichenden Therapieerfolg bzw. zu mitunter erheblichen Nebenwirkungen.

Vor diesem Hintergrund besteht aufgrund der Schwere und der Verbreitung der zuvor genannten Hautkrankheiten, insbesondere der Psoriasis bzw. des atopischen Ekzems, ein großer Bedarf, geeignete pharmazeutische Zusammensetzungen und Therapieformen bereitzustellen.

Somit liegt der vorliegenden Erfindung das Problem zugrunde, eine Zusammensetzung, insbesondere pharmazeutische Zusammensetzung, bereitzustellen, welche sich zur Behandlung von Hauterkrankungen, insbesondere zur Behandlung von Psoriasis bzw. des atopischen Ekzems (Neurodermitis), eignet, und welche die zuvor geschilderten Nachteile des Standes der Technik zumindest weitgehend vermeidet oder zumindest abschwächt. Dabei soll insbesondere eine verbesserte Wirksamkeit bei gleichzeitig verringerten Nebenwirkungen gewährleistet sein.

Die Anmelderin hat nunmehr in überraschender Weise herausgefunden, daß die zuvor geschilderte Aufgabe dadurch gelöst werden kann, daß man Ectoin bzw. mindestens ein Ectoinderivat zusammen mit Harnstoff bzw. mindestens einem Harnstoffderivat und/oder mindestens einem Inhaltsstoff des Johanniskrauts (*Hypericum perforatum L.*) im Rahmen einer Zusammensetzung, insbesondere einer pharmazeutischen Zusammensetzung, kombiniert bzw. appliziert bzw. formuliert.

Gegenstand der vorliegenden Erfindung - gemäß einem **ersten** Aspekt der vorliegenden Erfindung - ist somit Zusammensetzung, insbesondere pharmazeutische Zusammensetzung, vorzugsweise für die insbesondere topische Behandlung von Hauterkrankungen, wobei die Zusammensetzung - in jeweils wirksamen, insbesondere pharmazeutisch wirksamen Mengen - eine Kombination von
(a) einer Ectoin und Hydroxyectoin enthaltenden Mischung in Mengen von 1 bis 8 Gew.-%, bezogen auf die Zusammensetzung, wobei das Ectoin/Hydroxyectoin-Verhältnis 70 : 30 bis 99 : 1 beträgt; und
(b) Dexpanthenol und/oder Pantothensäure und/oder deren Ester in Mengen von 0,1 bis 10 Gew.-%, bezogen auf die Zusammensetzung,
   zusammen mit
(c) Harnstoff in Mengen von 0,001 bis 15 Gew.-%, bezogen auf die Zusammensetzung; und/oder
(d) mindestens einem Inhaltsstoff des Johanniskrauts (*Hypericum perforatum L.*) in Mengen von 0,0001 bis 5 Gew.-%, bezogen auf die Zusammensetzung, wobei der mindestens eine Inhaltsstoff des Johanniskrauts (*Hypericum perforatum L.*) ausgewählt ist aus der Gruppe von Hyperforin, Hypericin und deren Mischungen,
umfasst.

Der Begriff "pharmazeutische Zusammensetzung" bzw. "pharmazeutische Zubereitung" oder dergleichen, wie er im Rahmen der vorliegenden Erfindung verwendet wird, ist sehr umfassend zu verstehen und bezeichnet nicht nur pharmazeutische Präparate bzw. Pharmazeutika als solche, sondern auch sogenannte Medizinprodukte, homöopathische Mittel, Kosmetika oder dergleichen.

Bei allen nachstehend genannten relativen bzw. prozentualen gewichtsbezogenen Mengenangaben ist zu beachten, daß diese im Rahmen der erfindungsgemäßen Zusammensetzung bzw. Kombination vom Fachmann derart auszuwählen sind, daß sie sich in der Summe unter Einbeziehung der Komponenten bzw. Inhaltsstoffe bzw. Zusatzstoffe bzw. Bestandteile bzw. Exzipienten, insbesondere wie nachfolgend definiert, stets um 100 Gew.-% ergänzen. Dies versteht sich für den Fachmann aber von selbst. Im übrigen gilt, daß der Fachmann anwendungsbezogen oder einzelfallbedingt von den nachfolgend aufgeführten Mengenangaben abweichen kann, ohne daß er den Rahmen der vorliegenden Erfindung verläßt.

Die Anmelderin hat überraschenderweise herausgefunden, daß durch die Kombination von Ectoin und Hydroxyectoin zusammen mit Harnstoff und mindestens einem Inhaltsstoff des Johanniskrauts (*Hypericum perforatum L.*) (z. B. in erfindungsgemäß besonders bevorzugter Weise Hyperforin und/oder Hypericin) sowie Dexpanthenol bzw. Pantothensäure bzw. deren Estern die zuvor geschilderte Aufgabenstellung in effizienter Weise gelöst wird und ein wirksames Therapeutikum für die Behandlung von Hautkrankheiten, insbesondere der eingangs genannten Art, wie Psoriasis bzw. atopischem Ekzem, bereitgestellt wird, welches darüber hinaus gut verträglich ist und zumindest im wesentlichen keine Nebenwirkungen verursacht.

Diesbezüglich hat die Anmelderin in völlig überraschender Weise herausgefunden, daß die Kombination von Ectoin bzw. einem Ectoinderivat mit Harnstoff bzw. mindestens einem Harnstoffderivat einerseits oder mit mindestens einem Inhaltsstoff des Johanniskrauts (*Hypericum perforatum L.*) andererseits zu einer synergistischen Wirkweise führt, welche über die Einzelwirkung der Monopräparate in signifikanter Weise hinausgeht. Zudem hat die Anmelderin völlig überraschend herausgefunden, daß die Wirkung der erfindungsgemäßen Zusammensetzung auf Basis von Ectoin bzw. mindestens einem Ectoinderivat und Harnstoff bzw. mindestens einem Harnstoffderivat zusätzlich gesteigert bzw. verbessert werden kann, wenn diese Zusammensetzung insbesondere im Rahmen einer ternären Kombination mindestens einen Inhaltsstoff des Johanniskrauts (*Hypericum perforatum L.*) enthält. Dabei ist es im Rahmen der vorliegenden Erfindung in eindrucksvoller Weise gelungen, eine hochwirksame Zusammensetzung mit gleichzeitig signifikant verringerten Nebenwirkungen bereitzustellen.

In diesem Zusammenhang kann die Wirkweise der erfindungsgemäßen Zusammensetzung - ohne sich auf diese Theorie festlegen zu wollen - derart begründet werden, daß das Ectoin sich gewissermaßen wie eine Schutzschicht auf die Hautzellen legt und somit deren Austrocknung verhindert. Dies ist besonders wichtig, wenn die Haut geschädigt ist und durch Kratzeffekte an der Oberfläche verletzt ist. Unbehandelt kommt es dann bei diesen Vorschädigungen zu einer Steigerung des Juckreizes, zur bakteriellen Fehlbesiedlung und letztendlich zu einer Aggravierung des Krankheitsbildes. Ectoin bzw. dessen Derivate schützen die Hautzelle vor weiteren Schäden, indem es sich oberflächenmäßig auf die Hautzellen legt und diese gegen äußere Einflüsse sozusagen abschirmt.

Was den Harnstoff anbelangt, so führt dieser im Rahmen der erfindungsgemäß bevorzugten topischen Anwendung der Zusammensetzung nach der Erfindung auf der Haut dazu, daß - ohne sich auf diese Theorie festlegen zu wollen - das Wasser in den Zellen gehalten und so die Haut zusätzlich vor dem Austrocknen geschützt wird. Dies ist gleichermaßen eine wichtige Voraussetzung dafür, daß krankhafte Veränderungen an der Haut erst gar nicht entstehen bzw. bereits vorhandene Hautveränderungen schneller abheilen können.

Die Anmelderin hat nun überraschend gefunden, daß die beiden Effekte zusammen - also die Generierung einer Schutzschicht durch das Ectoin bzw. dessen Derivate einerseits und der durch den Harnstoff induzierte Schutz der Haut vor dem Austrocknen andererseits - in synergistischer Weise zusammenwirken und somit eine deutliche Steigerung der Wirksamkeit der erfindungsgemäßen Zusammensetzung im Vergleich zu den Monopräparaten des Standes der Technik resultiert. Die synergisitische Kombination von Ectoin bzw. dessen Derivaten einerseits zusammen mit Harnstoff bzw. dessen Derivaten andererseits stellt somit eine neue Potenzierung im Hautschutz dar, der besonders wichtig bei der Behandlung der Haut ist, wie z. B. im Rahmen der Behandlung der atopischen Dermatitis (Neurodermitis) oder Psoriasis.

Der bzw. die gegebenenfalls in der erfindungsgemäßen Zusammensetzung vorhandenen Inhaltsstoffe des Johanniskrauts (*Hypericum perforatum L.*) unterstützen die Wirkung weiterführend, da der bzw. die Inhaltsstoffe des Johanniskrauts (*Hypericum perforatum L.*) in der lokalen Applikation - ohne sich auf diese Theorie festlegen zu wollen - oberflächenadstringierende und oberflächendesinfizierende Eigenschaften aufweisen, die gerade auf gereizter Haut sinnvoll sind, da sie eine mögliche Fehlbesiedlung der Epidermis, beispielsweise mit *Streptococcus aureus,* eindämmen bzw. verhindern können.

Neben den vorgenannten Wirk- bzw. Inhaltsstoffen enthält die erfindungsgemäße Zusammensetzung außerdem mindestens einen pharmakologisch verträglichen Träger bzw. Exzipienten, welche dem Fachmann in ihrer Gesamtheit als solche wohlbekannt sind.

Bei der im Rahmen der vorliegenden Erfindung verwendeten Wirksubstanz Ectoin handelt es sich um (4S)-2-Methyl-1,4,5,6-tetrahydropyrimidin-4-carboxysäure gemäß der folgenden Strukturformel:

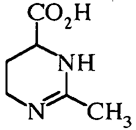

Bei dem erfindungsgemäß verwendeten Ectoin handelt es sich um einen zu der Gruppe der kompatiblen Solute gehörenden Naturstoff, welcher über stark wasserbindende Eigenschaften verfügt. Ectoin tritt in halo- bzw. extremophilen Bakterien auf. Ohne sich auf diese Theorie festlegen zu wollen, stabilisiert Ectoin die natürliche Struktur von Biopolymeren, wie Proteinen, Nukleinsäuren und Biomembranen, und schützt die Haut vor Schäden durch Streßfaktoren, wie Trockenheit, Hitze oder Kälte. Dabei sorgt Ectoin für einen nachhaltigen Feuchtigkeitsaufbau in der Haut. Für weitergehende diesbezügliche Ausführungen in bezug auf Ectoin, insbesondere in bezug auf dessen Herstellung, kann verwiesen werden auf die europäische Patentschrift EP 0 887 418 B1, deren gesamter diesbezüglicher Offenbarungsgehalt hiermit durch Bezugnahme eingeschlossen ist.

Erfindungsgemäß ist es zudem bevorzugt, wenn erfindungsgemäß ein sogenanntes Med-Ectoin eingesetzt wird, welches kommerziell erhältlich ist. Hierbei handelt es sich um ein hochreines Medizinprodukt, welches aus extremophilen Bakterien gewonnen wird und welches einen Gesamtectoingehalt von mehr als 99 % aufweist. Med-Ectoin enthält vorzugsweise mindestens 96 Gew.-% Ectoin und höchstens 4 Gew.-% Hydroxyectoin. Aufgrund der sehr hohen Reinheit ist Med-Ectoin sonders gut verträglich und weist ein definiertes Wirkspektrum auf.

Erfindungsgemäß ist es vorgesehen, daß die Komponente (a) in Form einer Ectoin und Hydroxyectoin enthaltenden Mischung vorliegt. Im Rahmen der vorliegenden Erfindung ist es somit vorgesehen, in bezug auf die Wirksubstanz (a) eine Mischung aus Ectoin und Hydroxyectoin einzusetzen, wobei das Ectoin/Hydroxyectoin-Verhältnis 70 : 30 bis 99 : 1, insbesondere 80 : 20 bis 95 : 5, betragen kann.

Was die Menge an Ectoin und Hydroxyectoin enthaltender Mischunganbelangt, so kann diese in weiten Bereichen variieren. Erfindungsgemäß enthält die erfindungsgemäße Zusammensetzung die Ectoin und Hydroxyectoin enthaltende Mischung in Mengen von 1 bis 8 Gew.-%, ganz besonders bevorzugt 2 bis 6 Gew.-%, bezogen auf die Zusammensetzung. Dennoch kann es im Einzelfall oder anwendungsbezogen erforderlich sein, von den vorgenannten Mengenangaben abzuweichen, ohne daß der Fachmann den Rahmen der vorliegenden Erfindung verläßt.

Was den Harnstoff anbelangt, so kann dessen Menge gleichermaßen in weiten Bereichen variieren: Erfindungsgemäß enthält die erfindungsgemäße Zusammensetzung Harnstoff in Mengen von 0,001 bis 15 Gew.-%, insbesondere 0,1 bis 10 Gew.-%, vorzugsweise 0,5 bis 7 Gew.-%, besonders bevorzugt 1 bis 5 Gew.-%, ganz besonders bevorzugt 1 bis 4 Gew.-%, bezogen auf die Zusammensetzung. Dennoch kann es im Einzelfall oder anwendungsbezogen erforderlich sein, von den vorgenannten Mengenangaben abzuweichen, ohne daß der Fachmann den Rahmen der vorliegenden Erfindung verläßt.

Für weitergehende Einzelheiten zu Harnstoff, welcher synonym auch als *Urea pura* bezeichnet wird, kann auf Römpp Chemielexikon, 10. Auflage, Band 3, 1997, Georg Thieme Verlag Stuttgart/New York, Seiten 1686/1687, Stichwort: "Harnstoff', sowie auf die dort referierte Literatur verwiesen werden, wobei der gesamte Offenbarungsgehalt der dort genannten Literatur hiermit durch Bezugnahme eingeschlossen ist.

Was den oder die Inhaltsstoffe des Johanniskrauts (*Hypericum perforatum L.*) anbelangt, so können diese in Form von Johanniskrautextrakt der erfindungsgemäßen Zusammensetzung zugesetzt sein. Die Herstellung von Johanniskrautextrakt ist dem Fachmann an sich bekannt, so daß es diesbezüglich keiner weiterführenden Ausführungen bedarf.

Was die Menge an Inhaltsstoff(en) des Johanniskrauts (*Hypericum perforatum L*.), insbesondere Johanniskrautextrakt, anbelangt, so kann auch diese in weiten Grenzen variieren. Erfindungsgemäß enthält die Zusammensetzung Inhaltsstoff(e) des Johanniskrauts (*Hypericum perforatum L.*), insbesondere Johanniskrautextrakt, in Mengen von 0,0001 bis 5 Gew.-%, insbesondere 0,001 bis 4 Gew.-%, vorzugsweise 0,01 bis 3 Gew.-%, besonders bevorzugt 0,1 bis 2 Gew.-%, bezogen auf die Zusammensetzung. Dennoch kann es im Einzelfall oder anwendungsbezogen erforderlich sein, von den vorgenannten Mengenangaben abzuweichen, ohne daß der Fachmann den Rahmen der vorliegenden Erfindung verläßt.

Erfindungsgemäß können der oder die Inhaltsstoff(e) des Johanniskrauts (*Hypericum perforatum L.*) aus der Gruppe von Hyperforin, Hypericin und deren Mischungen ausgewählt sein.

Gemäß einer erfindungsgemäß bevorzugten Ausführungsform werden der oder die Inhaltsstoffe des Johanniskrauts (*Hypericum perforatum L.*) oder Johanniskrautextrakt eingesetzt, wobei der Johanniskrautextrakt 200 bis 100.000 µg/ml, bevorzugt etwa 1.000 µg/ml Hyperforin und/oder 200 bis 2.000 µg/ml Hypericin enthält. Mit anderen Worten kann die erfindungsgemäße Zusammensetzung die aus Johanniskraut gewonnenen Wirksubstanzen Hyperforin und/oder Hypericin enthalten. Erfindungsgemäß ist es gleichermaßen möglich, daß die erfindungsgemäße Zusammensetzung nach der Erfindung pharmakologisch wirksame Derivate von Hypericin und/oder Hyperforin enthält.

Bei Hypericin handelt es sich um ein Antrachinonderivat, welches über antibiotische Eigenschaften verfügt. Hypericin wird synonym auch als Hypericumrot, Johannisblut bzw. Herrgottsblut bezeichnet. Für weitere diesbezügliche Einzelheiten zu Hypericin kann verwiesen werden auf Römpp Chemielexikon, 10. Auflage, Band 3, 1997, Georg Thieme Verlag Stuttgart/New York, Seiten 1860/1861, Stichwort: "Hypericin", sowie auf die dort referierte Literatur, wobei der gesamte Offenbarungsgehalt der dort genannten Literatur hiermit durch Bezugnahme eingeschlossen ist. Bei Hyperforin handelt es sich um einen sekundären Pflanzenstoff, welcher sich chemisch-strukturell durch Polyprenylierung von Phloroglucin ableiten läßt.

Was den erfindungsgemäß einsetzbaren Johanniskrautextrakt weiterhin anbelangt, so kann dieser Alkohol, insbesondere Ethanol, enthalten, wobei die Menge an Alkohol vorzugsweise 20 bis 60 Gew.-%, bezogen auf den Johanniskrautextrakt, betragen kann. Erfindungsgemäß ist es bevorzugt, einen flüssigen, wäßrigen, alkoholischen oder wäßrig-alkoholischen Extrakt einzusetzen. Grundsätzlich ist es im Rahmen der vorliegenden Erfindung aber auch möglich, den Johanniskrautextrakt in Form eines Trockenextraktes einzusetzen.

Neben den relativen bzw. prozentualen Mengenanteilen der Wirk- bzw. Inhaltsstoffe in der erfindungsgemäßen Zusammensetzung spielt auch das Mengenverhältnis (Gewichtsverhältnis) der Wirksubstanzen zueinander eine entscheidende Rolle in bezug auf die Wirksamkeit der erfindungsgemäßen Zusammensetzung. So ist es im Rahmen der vorliegenden Erfindung gelungen, die Wirksamkeit der erfindungsgemäßen Zusammensetzung in bezug auf die Behandlung von Hautkrankheiten, insbesondere der vorgenannten Art, durch eine gezielte Abstimmung des Mengenverhältnisses zwischen Ectoin bzw. dessen Derivat(en) einerseits und Harnstoff bzw. dessen Derivat(en) bzw. Inhaltsstoff(en) des Johanniskrauts (*Hypericum perforatum L.*) andererseits weiter zu erhöhen.

Im allgemeinen liegt das Mengenverhältnis (Gewichtsverhältnis) von Ectoin und/oder dem mindestens einen Ectoinderivat einerseits zu Harnstoff und/oder dem mindestens einen Harnstoffderivat andererseits in der Zusammensetzung im Bereich von 1 : 1.000 bis 1.000 : 1, insbesondere 1 : 100 bis 100 : 1, vorzugsweise 1 : 20 bis 20 : 1, bevorzugt 1 : 5 bis 5 : 1, ganz besonders bevorzugt etwa 2,3 : 1, berechnet als Ectoin zu Harnstoff.

Für den Fall, daß die erfindungsgemäße Zusammensetzung mindestens einen Inhaltsstoff des Johanniskrauts (*Hypericum perforatum L.*) enthält, kann das Mengenverhältnis (Gewichtsverhältnis) von Ectoin und/oder dem mindestens einen Ectoinderivat einerseits zu Inhaltsstoff(en) des Johanniskrauts (*Hypericum perforatum L.*) andererseits in der Zusammensetzung im Bereich von 1 : 5.000 bis 10.000 : 1, insbesondere 1 : 50 bis 5.000 : 1, vorzugsweise 1 : 10 bis 500 : 1, bevorzugt 1 : 2 bis 50 : 1, liegen, berechnet als Ectoin zu Inhaltsstoff des Johanniskrauts (*Hypericum perforatum L*.)*.*

Für den Fall, daß die erfindungsgemäße Zusammensetzung mindestens einen Inhaltsstoff des Johanniskrauts (*Hypericum perforatum L.*) enthält, kann in erfindungsgemäß bevorzugter Weise der Inhaltsstoff des Johanniskrauts (*Hypericum perforatum L.*) Hyperforin sein, wobei dieser in Mengen von 0,0001 bis 100 mg/ml der Zusammensetzung, insbesondere 0,02 bis 20 mg/ml der Zusammensetzung, besonders bevorzugt 1 bis 20 mg/ml der Zusammensetzung, eingesetzt werden. Dennoch kann es im Einzelfall oder anwendungsbezogen erforderlich sein, von den vorgenannten Mengenangaben abzuweichen, ohne daß der Fachmann den Rahmen der vorliegenden Erfindung verläßt.

Gemäß einer erfindungsgemäß bevorzugten Ausführungsform weist die Zusammensetzung nach der Erfindung eine hochviskose bzw. pastöse Konsistenz auf. Insbesondere ist die erfindungsgemäße Zusammensetzung als Salbe, Creme, Paste, Gel, Lotion oder dergleichen formuliert. Diesbezüglich kann die Zusammensetzung eine übliche Salben-, Creme-, Pasten-, Gel- oder Lotionsgrundlage enthalten. Aufgrund der spezifischen Konsistenz der erfindungsgemäßen Zusammensetzung kann diese besonders gut, d. h. in gleichmäßiger Weise und ohne hohen Kraftaufwand auf die Haut appliziert werden. Hierdurch wird eine besonders gute Verteilung und lange Kontaktzeit mit dem zu behandelnden Hautareal erreicht. Zu weitergehenden Einzelheiten in bezug auf die Begriffe der Salbe, Creme, Paste, des Gels oder der Lotion kann auf die diesbezüglichen Stichwörter in Römpp Chemielexikon, 10. Auflage, Band 3, Georg Thieme Verlag Stuttgart/New York, verwiesen werden.

Wenn die erfindungsgemäße Zusammensetzung gemäß der erfindungsgemäß bevorzugten Ausführungsform als Salbe, Creme, Paste, Gel, Lotion oder dergleichen formuliert ist, enthält sie an sich übliche Salben-, Creme-, Pasten-, Gel- oder Lotionsgrundlagen. Dies ist dem Fachmann an sich geläufig, so daß es diesbezüglich keiner weitergehenden Ausführung bedarf. Als Salben-, Creme-, Pasten-, Gel- oder Lotionsgrundlage bezeichnet man im Rahmen der vorliegenden Erfindung insbesondere die zur Herstellung von Salben, Cremes, Pasten, Gelen, Lotionen oder dergleichen benötigten Grundstoffe ("Salbengrundstoffe"), welche in der Kosmetik auch als Basen oder als Fonds bezeichnet werden. Die Salbengrundlage soll als Carrier oder Vehikel die Penetration der Wirkstoffe fördern. Die verschiedenen Salbengrundlagen werden beispielsweise nach ihrer chemischen Zusammensetzung eingeteilt (z. B. Kohlenwasserstoffgele, Lipogele, Wachse, Hydrogele, organische Gele, Polyethylenglykolgele, Silicongele etc.). Die Grundlagen für Salben, Cremes, Pasten oder Gele können außerdem üblicherweise Hilfsstoffe, wie Emulgatoren, Antioxidantien, Konservierungsstoffe, Feuchthaltemittel und dergleichen, enthalten. Zu weitergehenden Einzelheiten zum Begriff der Salbengrundlage kann beispielsweise auf Römpp Chemielexikon, 10. Auflage, Band 5, Georg Thieme Verlag Stuttgart/New York, 1998, Seite 3909, Stichwort: "Salbengrundlage", sowie die dort referierte Literatur verwiesen werden, wobei der gesamte Offenbarungsgehalt dieser Textstellen hiermit durch Bezugnahme eingeschlossen ist.

Die erfindungsgemäße Zusammensetzung kann zudem Allantoin bzw. dessen Derivate enthalten. Die diesbezüglichen Mengen können im Bereich von 0,001 bis 5 Gew.-%, insbesondere 0,01 bis 2 Gew.-%, vorzugsweise 0,1 bis 1 Gew.-%, liegen, bezogen auf die Zusammensetzung. Allantoin kann die Wirkung der erfindungsgemäßen Zusammensetzung unterstützen bzw. steigern, insbesondere da es eine Beschleunigung des Zellaufbaus, der Zellbildung bzw. der Zellregeneration bewirkt und die Haut gewissermaßen beruhigt. Auch die Heilung schwerheilender Wunden wird unterstützt, was insbesondere im Rahmen der erfindungsgemäß zu behandelnden Hauterkrankungen von besonders großem Vorteil ist.

Weiterhin enthält die erfindungsgemäße Zusammensetzung außerdem Dexpanthenol bzw. Pantothensäure bzw. deren Ester. Die diesbezüglichen Mengen liegen im Bereich von 0,1 bis 10 Gew.-%, insbesondere 0,5 bis 7 Gew.-%, vorzugsweise 1 bis 5 Gew.-%, bezogen auf die Zusammensetzung. Bei Dexpanthenol handelt es sich um den internationalen Freinamen für D-(+)-2,4-Dihydroxy-N-(3-hydroxypropyl)-3,3-dimethylbutylamid. Hierbei handelt es sich um eine wundheilend wirkende Substanz, welche die Wirkung der erfindungsgemäßen Zusammensetzung unterstützen bzw. steigern kann, beispielsweise durch ein verbessertes Ausheilen von Läsionen der Haut.

Darüber hinaus kann die erfindungsgemäße Zusammensetzung außerdem mindestens einen weiteren Wirk- bzw. Inhaltsstoff enthalten. Dieser kann insbesondere ausgewählt sein aus der Gruppe von Antiseptika, Lokalanästhetika, Vitaminen, Spurenelementen, Mineralien, Mikronährstoffen sowie deren Kombinationen. Der Fachmann ist jederzeit in der Lage, sowohl die konkreten Substanzen als auch die diesbezüglich zu wählenden Mengen in bezug auf die erfindungsgemäße Zusammensetzung auszuwählen bzw. anzupassen.

Schließlich kann die Zusammensetzung nach der Erfindung außerdem mindestens einen üblichen pharmazeutischen Zusatz- bzw. Hilfsstoff enthalten. Dieser kann insbesondere ausgewählt sein aus der Gruppe von Verarbeitungshilfsmitteln, Stabilisatoren, Emulgatoren, Antioxidantien, Konservierungsstoffen, Feuchthaltemitteln, pH-Stellmitteln, pH-Puffersubstanzen, Verdickungsmitteln, Färbe-, Puffer-, Riech-, Duft-, Streck-, Binde-, Netz- bzw. Konservierungsstoffen sowie deren Kombinationen. Der Fachmann ist jederzeit in der Lage, sowohl die konkreten Substanzen als auch die diesbezüglich zu wählenden Mengen in bezug auf die erfindungsgemäße Zusammensetzung auszuwählen bzw. anzupassen.

Gemäß einer erfindungsgemäß besonders bevorzugten Ausführungsform umfaßt die Zusammensetzung nach der Erfindung, insbesondere die erfindungsgemäße pharmazeutische Zusammensetzung, vorzugsweise für die insbesondere topische Behandlung von Hauterkrankungen, - in jeweils wirksamen, insbesondere pharmazeutisch wirksamen Mengen - eine Kombination von
(a) einer Ectoin und Hydroxyectoin enthaltenden Mischung in Mengen von 1 bis 8 Gew.-%, bezogen auf die Zusammensetzung, wobei das Ectoin/Hydroxyectoin-Verhältnis 70 : 30 bis 99 : 1 beträgt, zusammen mit
(b) Harnstoff in Mengen von 0,001 bis 15 Gew.-%, insbesondere 0,1 bis 10 Gew.-%, vorzugsweise 0,5 bis 7 Gew.-%, besonders bevorzugt 1 bis 5 Gew.-%, bezogen auf die Zusammensetzung; und
(c) mindestens einem Inhaltsstoff des Johanniskrauts (*Hypericum perforatum L.*), insbesondere Johanniskrautextrakt, in Mengen von 0,0001 bis 5 Gew.-%, insbesondere 0,001 bis 4 Gew.-%, vorzugsweise 0,01 bis 3 Gew.-%, besonders bevorzugt 0,1 bis 2 Gew.-%, bezogen auf die Zusammensetzung, insbesondere wobei der oder die Inhaltsstoff(e) des Johanniskrauts (*Hypericum perforatum L*.) ausgewählt sind aus der Gruppe von Hyperforin, Hypericin und deren Mischungen; und
(d) gegebenenfalls Allantoin und/oder dessen Derivaten, vorzugsweise in Mengen von 0,001 bis 5 Gew.-%, insbesondere 0,01 bis 2 Gew.-%, vorzugsweise 0,1 bis 1 Gew.-%, bezogen auf die Zusammensetzung; und
(e) Dexpanthenol und/oder Pantothensäure und/oder deren Estern in Mengen von 0,1 bis 10 Gew.-%, insbesondere 0,5 bis 7 Gew.-%, vorzugsweise 1 bis 5 Gew.-%, bezogen auf die Zusammensetzung.

Wie zuvor erörtert, eignet sich die erfindungsgemäße Zusammensetzung zur prophylaktischen und/oder therapeutischen Behandlung von Hauterkrankungen, insbesondere von Psoriasis und/oder atopischem Ekzem bzw. Neurodermitis. In diesem Zusammenhang kann die erfindungsgemäße Zusammensetzung topisch und/öder lokal auf die Hautstelle aufgebracht werden, wobei im Rahmen der vorliegenden Erfindung auch eine großflächige Anwendung möglich ist. Wie nachfolgend noch erörtert, kann die erfindungsgemäße Zusammensetzung sowohl im Rahmen einer Therapie bei akuten Erscheinungsformen der Psoriasis bzw. der Neurodermitis eingesetzt werden als auch im Rahmen einer Nachbehandlung bzw. Rezidivprophylaxe bzw. vorbeugenden Behandlung.

Was die Zusammensetzung nach der Erfindung weiterhin anbelangt, so liegt diese vorzugsweise in Behältnissen, insbesondere dosierfertig, eingebracht vor, wobei diesbezüglich Volumengrößen von 1 bis 500 ml, insbesondere 5 bis 250 ml, je Behältnis bzw. Applikationseinheit (Dosiereinheit) vorgesehen sein können.

Die vorliegende Erfindung betrifft - gemäß einem **weiteren** Aspekt der vorliegenden Erfindung - gleichermaßen ein Behältnis, welches die erfindungsgemäße Zusammensetzung enthält. Das erfindungsgemäße Behältnis, welches die Zusammensetzung nach der Erfindung enthält, kann insbesondere in Form einer Tube, Dose, Flasche, eines Pump- bzw. Dosierspenders oder dergleichen ausgebildet sein. Diesbezüglich kann es vorgesehen sein, daß das Behältnis in Form eines zur einmaligen Anwendung bestimmten, eine Einzeldosis der erfindungsgemäßen Zusammensetzung aufweisenden Behältnisses ausgebildet ist. Gleichermaßen ist es jedoch möglich, daß das erfindungsgemäße Behältnis gewissermaßen als Vorratsbehältnis zur Aufnahme eines größeren Volumens der erfindungsgemäßen Zusammensetzung ausgebildet ist. Insbesondere in diesem Fall kann das erfindungsgemäße Behältnis mit einer vorzugsweise wiederverschließbaren Verschlußvorrichtung ausgestattet sein.

Darüber hinaus eignet sich die zuvor beschriebene Zusammensetzung nach der vorliegenden Erfindung zur Verwendung im Bereich der Kosmetik. Wie nachfolgend noch erörtert, kann die erfindungsgemäße Zusammensetzung in diesem Zusammenhang zur prophylaktischen und/oder therapeutischen Behandlung der atopischen Dermatitis oder Psoriasis bzw. von weiteren Erkrankungen der Haut eingesetzt werden.

Darüber hinaus ist die erfindungsgemäße Zusammensetzung zur Verwendung bei der prophylaktischen und/oder therapeutischen Behandlung von Hauterkrankungen geeignet.

Bei der Hauterkrankung kann es sich - wie bereits geschildert - um eine atopische Dermatitis, welche synonym auch als Neurodermitis, atopisches Ekzem oder endogenes Ekzem bezeichnet wird, handeln. Insbesondere kann es sich bei der Hauterkrankung um eine subakute, akute oder chronische atopische Dermatitis handeln. Mit anderen Worten zielt die vorliegende Erfindung insbesondere auf die prophylaktische und/oder therapeutische Behandlung von Hautveränderungen ab, beispielsweise in Form von Ekzemen, welche in Zusammenhang mit dem atopischen Ekzem bzw. der atopischen Dermatitis stehen.

Darüber hinaus kann die erfindungsgemäße Zusammensetzung auch zur prophylaktischen und/oder therapeutischen Behandlung von Psoriasis, insbesondere Psoriasis guttata oder Psoriasis vulgaris, wie Psoriasis vulgaris vom Plaquetyp, verwendet werden.

Wie zuvor geschildert, wird durch die Verwendung der erfindungsgemäßen Zusammensetzung in bezug auf die vorgenannten Hauterkrankungen ein besonders guter Effekt erreicht, so daß eine deutliche Abnahme der mit der Hauterkrankung in Verbindung stehenden Symptome resultiert. Dies ist - wie zuvor erörtert - insbesondere auf die synergistische Wirkung basierend auf Ectoin bzw. dessen Derivat(en) einerseits und Harnstoff bzw. dessen Derivat(en) andererseits zurückzuführen. Denn aufgrund der erfindungsgemäßen Kombination wird insbesondere ein Austrocknen der Haut in effektiver Weise vermindert bzw. verhindert und zusätzlich eine Schutzschicht in bezug auf die Hautzellen etabliert, was insgesamt zu einem schnelleren Abheilen der Symptome führt bzw. im Rahmen einer prophylaktischen Anwendung die Entstehung der Symptome gewissermaßen im Ansatz verhindert bzw. verringert. Die synergistische Wirkweise auf Basis von Ectoin einerseits und Harnstoff andererseits kann durch die Gegenwart von Inhaltsstoffen aus Johanniskraut (beispielsweise in Form von Johanniskrautextrakt), insbesondere der vorgenannten Art, weiter gesteigert werden, da die Wirksubstanzen des Johanniskrauts über oberflächenadstringierende und damit leicht oberflächendesinfizierende Eigenschaften verfügen, so daß die Gesamtwirkung der erfindungsgemäßen Zusammensetzung nochmals deutlich gesteigert werden kann, insbesondere da eine Infektion der vorgeschädigten Haut mit unerwünschten Bakterien bzw. Erregern wirksam eingedämmt bzw. verhindert wird. Auch die erfindungsgemäße Kombination von Ectoin bzw. mindestens einem Ectoinderivat einerseits mit mindestens einem Inhaltsstoff aus Johanniskraut führt in überraschender Weise zu einem synergistischen Effekt in bezug auf die Wirkung der Zusammensetzung. Zur Vermeidung unnötiger Wiederholungen kann auf den zuvor in bezug auf die erfindungsgemäße Zusammensetzung beschriebenen Wirkmechanismus verwiesen werden.

Darüber hinaus kann die erfindungsgemäße Zusammensetzung auch zur prophylaktischen und/oder therapeutischen Behandlung von Ekzemen, insbesondere chronischen bis superinfizierten Ekzemen, Exsikationsekzemen, insbesondere hyperatotischen Hand- und Fußekzemen, Kontaktekzemen; Lichen, insbesondere Lichen simplex; Prurigo, insbesondere Prurigo simplex subacuta, verwendet werden.

Wie zuvor erörtert, kann die erfindungsgemäße Zusammensetzung auch zur Nachbehandlung und Rezidivprophylaxe der vorgenannten Hauterkrankungen eingesetzt werden.

Zu Zwecken der erfindungsgemäßen Verwendung kann die erfindungsgemäße Zusammensetzung topisch bzw. lokal auf die Haut appliziert werden, beispielsweise durch Einreiben bzw. Auftragen auf die zu behandelnden Hautareale. Diesbezüglich kann es vorgesehen sein, daß die erfindungsgemäße Zusammensetzung mindestens einmal täglich, vorzugsweise mehrmals täglich auf die Hautstellen appliziert wird. Aufgrund der sehr guten Verträglichkeit der erfindungsgemäßen Zusammensetzung kann die Applikation sowohl großflächig als auch über einen längeren Zeitraum erfolgen.

Die vorliegende Erfindung weist gegenüber dem Stand der Technik zahlreiche Vorteile auf, welche nachfolgend beispielhaft aufgeführt sind:
- Durch die gleichzeitige Verwendung von Ectoin bzw. einem Ectoinderivat einerseits zusammen mit Harnstoff bzw. einem Harnstoffderivat andererseits wird gewissermaßen eine Doppelstrategie realisiert: Durch die Verwendung von Ectoin bzw. dessen Derivaten wird gewissermaßen eine Schutzschicht auf die Hautzellen gelegt, was eine Austrocknung der Haut deutlich verringert. Durch den Einsatz von Harnstoff wird die Austrocknung weiter herabgesetzt, was einerseits dazu führt, daß bestehende Symptome rasch abklingen bzw. krankhafte Veränderungen an der Haut erst gar nicht entstehen können.
- Aufgrund der zusätzlichen Verwendung von mindestens einem Inhaltsstoff des Johanniskrauts (*Hypericum perforatum L.*) kann die Wirkung bzw. Effektivität der erfindungsgemäßen Zusammensetzung auf Basis von Ectoin und/oder Harnstoff noch weiter gesteigert werden, da aufgrund der leicht oberflächendesinfizierenden Eigenschaften der Inhaltsstoffe von Johanniskraut eine Fehlbesiedlung bzw. Infektion der Haut mit schädlichen Erregern, insbesondere vom *Streptococcus-Typ,* vermieden wird.
- Die erfindungsgemäße Zusammensetzung ist gut verträglich und weist keine bzw. nur sehr geringe Nebenwirkungen auf, so daß die erfindungsgemäße Zusammensetzung sowohl großflächig auf die Haut appliziert werden kann als auch über einen längeren Zeitraum angewendet werden kann.
- Aufgrund der guten Wirkung der erfindungsgemäßen Zusammensetzung heilen die Beschwerden rasch ab, so daß der Leidensdruck der von den Hautkrankheiten der vorgenannten Art betroffenen Patienten deutlich verringert wird. Insbesondere werden die betroffenen Patienten von dem oftmals quälenden Juckreiz befreit, was zu einer drastischen Verbesserung der Lebenssituation führt.
- Bei den im Rahmen der erfindungsgemäßen Zusammensetzung verwendeten Wirksubstanzen handelt es sich um natürliche bzw. naturidentische Stoffe, die dazu führen, daß die erfindungsgemäße Zusammensetzung im wesentlichen frei von Nebenwirkungen ist.

Weitere Ausgestaltungen, Abwandlungen und Variationen sowie Vorteile der vorliegenden Erfindung sind für den Fachmann beim Lesen der Beschreibung ohne weiteres erkennbar und realisierbar, ohne daß er dabei den Rahmen der vorliegenden Erfindung verläßt.

### Ausführungsbeispiele:

Im Rahmen von seitens der Anmelderin durchgeführten Ausführungsbeispielen bzw. Wirksamkeitsstudien wurde die herausragende Wirkung einer Zusammensetzung nach der vorliegenden Erfindung auf Basis der erfindungsgemäßen Wirkstoffkombination auf Basis von Ectoin und Harnstoff (vgl. nachfolgende "Zusammensetzung C") gegenüber einer Vergleichszusammensetzung, welche entweder nur Harnstoff als Wirksubstanz (vgl. nachfolgende "Zusammensetzung A", nicht erfindungsgemäß) bzw. nur Ectoin als Wirksubstanz (vgl. nachfolgende "Zusammensetzung B", nicht erfindungsgemäß) belegt. In diesem Zusammenhang wurde die synergistische Wirkung der erfindungsgemäßen Kombination auf Basis von Ectoin einerseits und Harnstoff andererseits gezeigt, welche über die additive Wirkung der Einzelsubstanzen Harnstoff bzw. Ectoin hinausgeht. Zudem wurde eine nochmals verbesserte Wirksamkeit gezeigt, wenn der erfindungsgemäßen Kombination auf Basis von Ectoin und Harnstoff zusätzlich Johanniskrautextrakt zugegeben wurde (vgl. nachfolgende "Zusammensetzung D", erfindungsgemäß). Auch wurde die synergistische Wirkung einer erfindungsgemäßen Kombination auf Basis von Ectoin einerseits und Johanniskrautextrakt andererseits gezeigt, welche über die additive Wirkung der Einzelsubstanzen Johanniskrautextrakt bzw. Ectoin hinausgeht (vgl. nachfolgende "Zusammensetzung E").

Zu den Zusammensetzungen im einzelnen:

### Zusammensetzung A (Vergleich):

Zur Herstellung der Zusammensetzung A wurde zunächst eine Vorlage hergestellt, welche, bezogen auf die Vorlage,

| | |
|---|---|
| 5,0 Gew.-% | Med-Ectoin; |
| 0,3 Gew.-% | Allantoin; |
| 2,0 Gew.-% | Dexpanthenol und |
| 56,0 Gew.-% | Wasser |

enthielt. Die restlichen Bestandteile (auf 100 Gew.-%) waren Salbengrundlage sowie übliche Hilfsstoffe. Von der Vorlage wurde eine Menge von 93,0 Gewichtsteilen abgenommen und in diese Menge 7 Gewichtsteile warmes Wasser eingearbeitet.

### Zusammensetzung B (Vergleich):

Zur Herstellung der Zusammensetzung B wurde zunächst eine Vorlage hergestellt, welche, bezogen auf die Vorlage,

| | |
|---|---|
| 0,3 Gew.-% | Allantoin; |
| 2,0 Gew.-% | Dexpanthenol und |
| 61,0 Gew.-% | Wasser |

enthielt. Die restlichen Bestandteile (auf 100 Gew.-%) waren Salbengrundlage sowie übliche Hilfsstoffe. Von der Vorlage wurde eine Menge von 93,0 Gewichtsteilen abgenommen. In 5,0 Gewichtsteile warmes Wasser wurden 2,0 Gewichtsteile Harnstoff eingearbeitet. Dieser Ansatz aus Wasser und Harnstoff wurde in 93,0 Gewichtsteile der vorgenannten Vorlage eingearbeitet.

### Zusammensetzung C:

Zur Herstellung der Zusammensetzung C wurde zunächst eine Vorlage hergestellt, welche, bezogen auf die Vorlage,

| | |
|---|---|
| 5,0 Gew.-% | Med-Ectoin; |
| 0,3 Gew.-% | Allantoin; |
| 2,0 Gew.-% | Dexpanthenol und |
| 56,0 Gew.-% | Wasser |

enthielt. Die restlichen Bestandteile (auf 100 Gew.-%) waren Salbengrundlage sowie übliche Hilfsstoffe. Von der Vorlage wurde eine Menge von 93,0 Gewichtsteilen abgenommen. In 5,0 Gewichtsteile warmes Wasser wurden 2,0 Gewichtsteile Harnstoff eingearbeitet. Dieser Ansatz aus Wasser und Harnstoff wurde in 93,0 Gewichtsteile der vorgenannten Vorlage eingearbeitet.

### Zusammensetzung D (Erfindung):

Die Zusammensetzung D entsprach der Zusammensetzung C unter Austausch von 1 Gew.-% Wasser in der Vorlage durch 1 Gew.-% Johanniskrautextrakt.

### Zusammensetzung E:

Die Zusammensetzung E entsprach der Zusammensetzung C unter Austausch der 2,0 Gewichtsteile Harnstoff durch dieselbe Menge an Johanniskrautextrakt.

### 1. Untersuchung der Hautrauhigkeit, Hautschuppigkeit sowie des transepidermalen Wasserverlustes

Im Rahmen einer ersten Untersuchungsreihe wurden insgesamt 21 Patienten mit diagnostizierter Neurodermitis einer Behandlung mit den zuvor genannten Zusammensetzungen unterzogen. Die jeweilige Größe der Untersuchungsgruppen für die Zusammensetzungen A bis C betrug n = 7. Die Zusammensetzungen wurden über einen Zeitraum von fünf Wochen dreimal täglich auf definierte Hautareale appliziert, welche die typischen Symptome einer Neurodermitis zeigten. Vor der ersten Applikation der Zusammensetzungen (Zeitpunkt t = 0) und nach Beendigung des Versuchs (Zeitpunkt t = 5 Wochen) wurde die Hautrauhigkeit sowie die Hautschuppigkeit mit computerunterstützten Auswerteverfahren nach anerkannten Methoden des Standes der Technik durch visuelle Begutachtung ermittelt und im Rahmen eines Schulnotensystems von 1 (geringe Hautrauhigkeit bzw. Hautschuppigkeit) bis 6 (sehr hohe Hautrauhigkeit bzw. Hautschuppigkeit) quantifiziert. Was die Ermittlung des transepidermalen Wasserverlustes (TEWL) anbelangt, so wurde dieser mit einer wissenschaftlich anerkannten und etablierten Meßvorrichtung ermittelt. Der Wert des transepidermalen Wasserverlustes vor Versuchsbeginn (Zeitpunkt t = 0) wurde gleich 100 % gesetzt. Die Prozentwerte nach Beendigung des Versuches (Zeitpunkt t = 5 Wochen) beziehen sich auf den 100 %-Wert zum Zeitpunkt t = 0. Die Werte für die Hautrauhigkeit und Hautschuppigkeit sowie die Werte für den transepidermalen Wasserverlust nach Versuchsende wurden gemittelt und die entsprechenden Standardabweichungen berechnet. Die Ergebnisse sind in der nachfolgenden Tabelle 1 angeführt.

**Tabelle 1:**

| | Zusammensetzung A (Ectoin) | | Zusammensetzung B (Harnstoff) | | Zusammensetzung C (Ectoin + Harnstoff) | |
|---|---|---|---|---|---|---|
| | t = 0 | t = 5 Wochen | t = 0 | t = 5 Wochen | t = 0 | t = 5 Wochen |
| Hautrauhigkeit | 5,2 ± 0,4 | 4,1 ± 0,2 | 5,5 ± 0,3 | 4,5 ± 0,3 | 5,4 ± 0,3 | 2,1 ± 0,2 |
| Hautschuppigkeit | 5,8 ± 0,2 | 3,8 ± 0,4 | 5,7 ± 0,2 | 4,2 ± 0,5 | 5,7 ± 0,4 | 1,7 ± 0,4 |
| transepidermaler Wasserverlust (TEWL) / % | 100 | 75,1 ± 5,1 | 100 | 80,5 ± 7,8 | 100 | 36,1 ± 8,0 |

Die Tabelle 1 veranschaulicht die hervorragende Wirkung der Zusammensetzung auf Basis der Kombination von Ectoin einerseits und Harnstoff andererseits gemäß Zusammensetzung C gegenüber den Vergleichszusammensetzungen (Zusammensetzung A und B), welche nur Ectoin oder Harnstoff enthielten.

Wie der Tabelle 1 zu entnehmen ist, werden sowohl die Hautrauhigkeit als auch die Hautschuppigkeit im Rahmen der Verwendung der Zusammensetzung C im Vergleich zu den Vergleichszusammensetzungen A und B signifikant reduziert. In diesem Zusammenhang wurde in bezug auf die Zusammensetzung C bei 5 Probanden eine nahezu vollständige Abheilung der von der Neurodermitis betroffenen Hautareale beobachtet, lediglich 2 Probanden wiesen allenfalls geringe Restsymptome auf. Dagegen konnte in bezug auf die Vergleichszusammensetzungen A und B eine allenfalls mäßige Abheilung beobachtet werden. In den meisten Fällen konnten nach der Behandlung weiterhin deutliche Symptome einer Neurodermitis manifestiert werden. Die Versuchreihe belegt somit in eindrucksvoller Weise die synergistische Wirkung der Zusammensetzung C auf Basis von Ectoin und Harnstoff.

Eine weitere Versuchsreihe nach obigem Schema mit 6 Patienten mit diagnostizierter Neurodermitis unter Verwendung einer erfindungsgemäßen Zusammensetzung auf Basis von Ectoin und Harnstoff und Johanniskrautextrakt (Zusammensetzung D) führte zu gegenüber der Zusammensetzung C nochmals verbesserten Ergebnissen (vgl. Tabelle 2).

Eine wiederum weitere Versuchsreihe nach obigem Schema mit 7 Patienten mit diagnostizierter Neurodermitis unter Verwendung einer Zusammensetzung auf Basis von Ectoin und Johanniskrautextrakt (Zusammensetzung E) führte zu gegenüber den Zusammensetzungen A und B verbesserten Ergebnissen (vgl. Tabelle 2).

**Tabelle 2:**

| | Zusammensetzung D (Ectoin + Harnstoff + Johanniskrautextrakt) | | Zusammensetzung E (Ectoin + Johanniskrautextrakt) | |
|---|---|---|---|---|
| | t = 0 | t = 5 Wochen | t = 0 | t = 5 Wochen |
| Hautrauhigkeit | 5,4 ± 0,2 | 1,7 ± 0,2 | 5,3 ± 0,2 | 2,4 ± 0,4 |
| Hautschuppigkeit | 5,6 ± 0,2 | 1,3 ± 0,2 | 5,8 ± 0,3 | 2,0 ± 0,3 |
| transepidermaler Wasserverlust (TEWL) / % | 100 | 29,5 ± 5,4 | 100 | 40,0 ± 6,3 |

### 2. Prick-Test/Atopie-Patch-Test:

Im Rahmen einer zweiten Versuchsreihe wurden insgesamt 15 Probanden mit diagnostizierter Neurodermitis zunächst einem Prick-Test unter Verwendung definierter Allergene unterzogen, wobei die Probanden zu Versuchsbeginn jedoch kein sichtbares Ekzem aufwiesen. Bei sämtlichen Probanden fiel der Prick-Test positiv aus. Anschließend wurde an allen Probanden ein Atopie-Patch-Test als Modell für die Auslösephase der Neurodermitis auf Basis wissenschaftlich anerkannter Methoden durchgeführt. Auch der Atopie-Patch-Test fiel bei sämtlichen Probanden positiv aus.

Die Probanden wurden in drei Versuchgruppen zu je fünf Personen aufgeteilt. Anschließend wurden die Zusammensetzungen A bis C gruppenspezifisch (Gruppe 1: Zusammensetzung A; Gruppe 2: Zusammensetzung B; Gruppe 3: Zusammensetzung C) über einen Zeitraum von fünf Wochen dreimal täglich auf definierte Hautareale appliziert.

Nach Beendigung der Behandlung wurde bei allen Versuchsteilnehmern erneut ein Atopie-Patch-Test durchgeführt. In bezug auf die Gruppe 1 (Vergleich, Zusammensetzung A) entwickelten drei Probanden mäßige Symptome einer Neurodermitis, bei zwei Probanden wurden leichte bis mäßige Symptome festgestellt. In bezug auf die Gruppe 2 (Vergleich, Zusammensetzung B) entwickelte ein Proband mäßige bis schwere Symptome einer Neurodermitis, während die übrigen vier Probanden mäßige Symptome aufwiesen. Hinsichtlich der Gruppe 3 (Zusammensetzung C) blieben zwei Probanden nach Durchführung des Atopie-Patch-Testes frei von Symptomen, bei zwei Probanden zeigten sich nur leichte Symptome, und bei einem Probanden wurden leichte bis mäßige Symptome ermittelt.

Somit belegt auch der Prick-Test/Atopie-Patch-Test die hervorragende, synergistische Wirkung der erfindungsgemäßen Kombination auf Basis von Ectoin und Harnstoff gemäß der Zusammensetzung C.

Bei weiteren insgesamt 12 Probanden mit diagnostizierter Neurodermitis mit positivem Prick-Test und positivem Atopie-Patch-Test wurden die Zusammensetzungen D und E gruppenspezifisch (Gruppe 4: Zusammensetzung D, 6 Probanden; Gruppe 5: Zusammensetzung E, 6 Probanden) über einen Zeitraum von fünf Wochen dreimal täglich auf definierte Hautareale appliziert. Nach Beendigung der Behandlung wurde bei allen Versuchsteilnehmern erneut ein Atopie-Patch-Test durchgeführt. In bezug auf die Gruppe 4 blieben vier Probanden nach Durchführung des Atopie-Patch-Testes frei von Symptomen, bei einem Probanden zeigten sich allenfalls nur leichte Symptome, und bei einem Proband wurden leichte bis mäßige Symptome beobachtet. In bezug auf die Gruppe 5 blieben drei Probanden nach Durchführung des Atopie-Patch-Testes frei von Symptomen, bei einem Probanden zeigten sich allenfalls nur leichte Symptome, und bei zwei Probanden wurden leichte bis mäßige Symptome beobachtet.

Somit belegt auch der Prick-Test/Atopie-Patch-Test die hervorragende, synergistische und gegenüber der Zusammensetzung C noch verbesserte Wirkung der erfindungsgemäßen Kombination auf Basis von Ectoin, Harnstoff und Johanniskrautextrakt gemäß der erfindungsgemäßen Zusammensetzung D, während im Fall der Zusammensetzung E gleichermaßen zufriedenstellende Ergebnisse erzielt werden konnten.

Die vorstehenden Versuchsreihen zeigen somit insgesamt die hervorragende, synergistische Wirkung der erfindungsgemäßen Kombination gegenüber den jeweiligen Kontrollexperimenten.

## Patentansprüche

1. Zusammensetzung, insbesondere pharmazeutische Zusammensetzung, vorzugsweise für die insbesondere topische Behandlung von Hauterkrankungen, wobei die Zusammensetzung - in jeweils wirksamen, insbesondere pharmazeutisch wirksamen Mengen - eine Kombination von
(a) einer Ectoin und Hydroxyectoin enthaltenden Mischung in Mengen von 1 bis 8 Gew.-%, bezogen auf die Zusammensetzung, wobei das Ectoin/Hydroxyectoin-Verhältnis 70 : 30 bis 99 : 1 beträgt; und
(b) Dexpanthenol und/oder Pantothensäure und/oder deren Ester in Mengen von 0,1 bis 10 Gew.-%, bezogen auf die Zusammensetzung,
zusammen mit
(c) Harnstoff in Mengen von 0,001 bis 15 Gew.-%, bezogen auf die Zusammensetzung; und/oder
(d) mindestens einem Inhaltsstoff des Johanniskrauts (*Hypericum perforatum L.*) in Mengen von 0,0001 bis 5 Gew.-%, bezogen auf die Zusammensetzung, wobei der mindestens eine Inhaltsstoff des Johanniskrauts (*Hypericum perforatum L*.) ausgewählt ist aus der Gruppe von Hyperforin, Hypericin und deren Mischungen,
umfasst.

2. Zusammensetzung nach Anspruch 1, enthaltend (a) die Ectoin und Hydroxyectoin enthaltende Mischung in Mengen von 2 bis 6 Gew.-%, bezogen auf die Zusammensetzung.

3. Zusammensetzung nach Anspruch 1 oder 2, enthaltend (c) Harnstoff in Mengen von 0,1 bis 10 Gew.-%, vorzugsweise 0,5 bis 7 Gew.-%, besonders bevorzugt 1 bis 5 Gew.-%, ganz besonders bevorzugt 1 bis 4 Gew.-%, bezogen auf die Zusammensetzung.

4. Zusammensetzung nach einem oder mehreren der vorangehenden Ansprüche, wobei (d) der oder die Inhaltsstoffe des Johanniskrauts (*Hypericum perforatum L.*) in Form von Johanniskrautextrakt zugesetzt sind.

5. Zusammensetzung nach einem oder mehreren der vorangehenden Ansprüche, enthaltend (d) Inhaltsstoff(e) des Johanniskrauts (*Hypericum perforatum L.*)*,* insbesondere Johanniskrautextrakt, in Mengen von 0,001 bis 4 Gew.-%, vorzugsweise 0,01 bis 3 Gew.-%, besonders bevorzugt 0,1 bis 2 Gew.-%, bezogen auf die Zusammensetzung.

6. Zusammensetzung nach einem oder mehreren der vorangehenden Ansprüche, wobei (d) der oder die Inhaltsstoffe des Johanniskrauts (*Hypericum perforatum L.*) oder Johanniskrautextrakt eingesetzt werden, wobei der Johanniskrautextrakt 200 bis 100.000 µg/ml, bevorzugt etwa 1.000 µg/ml Hyperforin und/oder 200 bis 2.000 µg/ml Hypericin enthält und/oder wobei der Johanniskrautextrakt Alkohol, insbesondere Ethanol, enthält, vorzugsweise in Mengen von 20 bis 60 Gew.-%, bezogen auf den Johanniskrautextrakt.

7. Zusammensetzung nach einem oder mehreren der vorangehenden Ansprüche, wobei der Inhaltsstoff des Johanniskrauts (*Hypericum perforatum L.*) Hyperforin ist und in Mengen von 0,0001 bis 100 mg/ml der Zusammensetzung, insbesondere 0,02 bis 20 mg/ml der Zusammensetzung, besonders bevorzugt 1 bis 20 mg/ml der Zusammensetzung, eingesetzt wird.

8. Zusammensetzung nach einem oder mehreren der vorangehenden Ansprüche, wobei die Zusammensetzung außerdem Allantoin enthält, vorzugsweise in Mengen von 0,001 bis 5 Gew.-%, insbesondere 0,01 bis 2 Gew.-%, vorzugsweise 0,1 bis 1 Gew.-%, bezogen auf die Zusammensetzung.

9. Zusammensetzung nach einem oder mehreren der vorangehenden Ansprüche, wobei die Zusammensetzung (b) Dexpanthenol und/oder Pantothensäure und/oder deren Ester in Mengen von 0,5 bis 7 Gew.-%, vorzugsweise 1 bis 5 Gew.-%, bezogen auf die Zusammensetzung, enthält.

10. Behältnis, enthaltend eine Zusammensetzung nach einem oder mehreren der vorangehenden Ansprüche, insbesondere in Form einer Tube, Dose, Flasche, eines Pump- und/oder Dosierspenders oder dergleichen.

11. Verwendung einer Zusammensetzung nach einem oder mehreren der Ansprüche 1 bis 9 zur Herstellung eines Arzneimittels zur prophylaktischen und/oder therapeutischen Behandlung von Hauterkrankungen.

## Claims

1. A composition, in particular a pharmaceutical composition, preferably for the particularly topical treatment of skin diseases, wherein the composition comprises - in effective amounts in each case, in particular in pharmaceutically effective amounts - a combination of
(a) a mixture containing ectoine and hydroxyectoine in amounts of 1 to 8 wt.%, based on the composition, wherein the ectoine/hydroxyectoine ratio is 70: 30 to 99: 1; and
(b) dexpanthenol and/or pantothenic acid and/or esters thereof in amounts of 0.1 to 10 wt.%, based on the composition,
together with
(c) urea in amounts of 0.001 to 15 wt.%, based on the composition; and/or
(d) at least one ingredient of St John's wort (*hypericum perforatum L.*) in amounts of 0.0001 to 5 wt.%, based on the composition, wherein the at least one ingredient of St John's wort (*hypericum perforatum L.*) is selected from the group of hyperforin, hypericin and mixtures thereof.

2. The composition according to claim 1, containing (a) the mixture containing ectoine and hydroxyectoine in amounts of 2 to 6 wt.%, based on the composition.

3. The composition according to claim 1 or 2, containing (c) urea in amounts of 0.1 to 10 wt.%, preferably 0.5 to 7 wt.%, particularly preferably 1 to 5 wt.%, very particularly preferably 1 to 4 wt.%, based on the composition.

4. The composition according to one or more of the preceding claims, wherein (d) the ingredient or ingredients of the St John's wort (*hypericum perforatum L.*) are added in the form of St John's wort extract.

5. The composition according to one or more of the preceding claims, containing (d) ingredient(s) of St John's wort (*hypericum perforatum L.*), in particular the St John's wort extract, in amounts of 0.001 to 4 wt.%, preferably 0.01 to 3 wt.%, particularly preferably 0.1 to 2 wt.%., based on the composition.

6. The composition according to one or more of the preceding claims, wherein (d) the ingredient or ingredients of St John's wort (*hypericum perforatum L.*) or St John's wort extract are used, wherein the St John's wort extract contains 200 to 100,000 µg/ml, preferably approximately 1000 µg/ml hyperforin and/or 200 to 2000 µg/ml hypericin and/or wherein the St John's wort extract contains alcohol, in particular ethanol, preferably in amounts of 20 to 60 wt.%, based on the St John's wort extract.

7. The composition according to one or more of the preceding claims, wherein the ingredient of St John's wort (*hypericum perforatum L.*) is hyperforin and is used in amounts of 0.0001 to 100 mg/ml of the composition, in particular 0.02 to 20 mg/ml of the composition, particularly preferably 1 to 20 mg/ml of the composition.

8. The composition according to one or more of the preceding claims, wherein the composition also contains allantoin, preferably in amounts of 0.001 to 5 wt.%, in particular 0.01 to 2 wt.%, preferably 0.1 to 1 wt.%, based on the composition.

9. The composition according to one or more of the preceding claims, wherein the composition (b) contains dexpanthenol and/or pantothenic acid and/or esters thereof in amounts of 0.5 to 7 wt.%, preferably 1 to 5 wt.%, based on the composition.

10. A container containing a composition according to one or more of the preceding claims, in particular in the form of a tube, a can, a bottle, a pump dispenser and/or metering dispenser or the like.

11. Use of a composition according to one or more of claims 1 to 9 for producing a medicament for the prophylactic and/or therapeutic treatment of skin diseases.

## Revendications

1. Composition, en particulier composition pharmaceutique, de préférence pour le traitement en particulier topique de maladies de la peau, la composition comprenant, dans chaque cas en les quantités actives, en particulier à activité pharmaceutique, une combinaison
(a) d'un mélange d'ectoïne et d'hydroxyectoïne en des quantités de 1 à 8 % en poids par rapport à la composition, le rapport ectoïne/hydroxyectoïne étant de 70 : 30 à 99 : 1 ; et
(b) du dexpanthénol et/ou de l'acide pentothénique et/ou des esters de celui-ci, en des quantités de 0,1 à 10 % en poids par rapport à la composition,
ainsi que
(c) de l'urée en des quantités de 0,001 à 15 % en poids, par rapport à la composition ; et/ou
(d) au moins un constituant du millepertuis (*Hypericum perforatum L.*) en des quantités de 0,0001 à 5 % en poids par rapport à la composition, le ou les constituants du millerpertuis (*Hypericum perforatum L.*) étant choisis dans le groupe de l'hyperforine, de l'hypericine et des mélanges de celles-ci.

2. Composition selon la revendication 1, contenant (a) le mélange contenant l'ectoïne et l'hydroxyectoïne en des quantités de 2 à 6 % en poids par rapport à la composition.

3. Composition selon la revendication 1 ou 2, contenant (c) de l'urée en des quantités de 0,1 à 10 % en poids, de préférence de 0,5 à 7 % en poids, d'une manière particulièrement préférée de 1 à 5 % en poids, d'une manière tout particulièrement préférée de 1 à 4 % en poids, par rapport à la composition.

4. Composition selon l'une ou plusieurs des revendications précédentes, dans laquelle (d) le ou les constituants du millepertuis (*Hypericum perforatum L.*) sont ajoutés sous forme d'extrait de millepertuis.

5. Composition selon l'une ou plusieurs des revendications précédentes, contenant (d) un ou plusieurs constituants du millepertuis (*Hypericum perforatum L.*)*,* en particulier de l'extrait de millepertuis, en des quantités de 0,001 à 4 % en poids, de préférence de 0,01 à 3 % en poids, d'une manière particulièrement préférée de 0,1 à 2 % en poids, par rapport à la composition.

6. Composition selon l'une ou plusieurs des revendications précédentes, dans laquelle on utilise (d) le ou les constituants du millepertuis (*Hypericum perforatum L.)* ou l'extrait de millepertuis, l'extrait de millepertuis contenant 200 à 100 000 µg/ml, de préférence environ 1000 µg/ml d'hyperforine et/ou 200 à 2000 µg/ml d'hypéricine, et/ou l'extrait de millepertuis contenant un alcool, en particulier l'éthanol, de préférence en des quantités de 20 à 60 % en poids, par rapport à l'extrait de millepertuis.

7. Composition selon l'une ou plusieurs des revendications précédentes, dans laquelle le constituant du millepertuis (*Hypericum perforatum L.)* est l'hyperforine et est utilisé en des quantités de 0,0001 à 100 mg/ml de la composition, en particulier de 0,02 à 20 mg/ml de la composition, d'une manière particulièrement préférée de 1 à 20 mg/ml de la composition.

8. Composition selon l'une ou plusieurs des revendications précédentes, la composition contenant en outre de l'allantoïne, de préférence en des quantités de 0,001 à 5 % en poids, en particulier de 0,01 à 2 % en poids, de préférence de 0,1 à 1 % en poids, par rapport à la composition.

9. Composition selon l'une ou plusieurs des revendications précédentes, la composition contenant (b) du dexpanthénol et/ou de l'acide pantothénique et/ou des esters de celui-ci, en des quantités de 0,5 à 7 % en poids, de préférence de 1 à 5 % en poids, par rapport à la composition.

10. Récipient contenant une composition selon l'une ou plusieurs des revendications précédentes, en particulier sous forme d'un tube, d'une boîte, d'un flacon, d'un distributeur à pompe et/ou d'un distributeur doseur ou analogues.

11. Utilisation d'une composition selon l'une ou plusieurs des revendications 1 à 9 pour la fabrication d'un médicament destiné au traitement prophylactique et/ou thérapeutique de maladies de la peau.
